# EUROPEAN PATENT APPLICATION

(11) **EP 4 706 504 A1**
(43) Date of publication of application: **11.03.2026**
(21) Application number: 24198862.5
(22) Date of filing: 06.09.2024
(51) Int. Cl.: A61B 5/00, A61B 5/18, A61B 5/113, A61B 5/08

(54) **METHOD, DEVICE AND SYSTEM FOR DETERMINING RESPIRATORY INFORMATION OF A SUBJECT**

(71) Applicant: Koninklijke Philips N.V., 5656 AG Eindhoven (NL)
(72) Inventor: VAREKAMP, Christiaan, Eindhoven (NL); VOSTERS, Luc Petrus Johannes, Eindhoven (NL); VAN GASTEL, Mark Josephus Henricus, 5656 AG Eindhoven (NL); DILLEN, Paulus Henricus Antonius, Eindhoven (NL)
(74) Representative: Philips Intellectual Property & Standards

(57) **Abstract**

The present invention relates to a method, device and system for determining respiratory information of a subject. The method obtains depth sensor signals relating to distances of a plurality of locations in a region of interest from a depth sensor. The region of interest includes at least a portion of the frontal torso of the subject. A variation in the distances of the plurality of locations in the region of interest and an average depth of the plurality of locations in the region of interest both based on the depth sensor signals acquired by the depth sensor at the first point in time, are determined. Based on the variation and the average depth in the region of interest, respiratory information is determined.

## Description

### FIELD OF THE INVENTION

The present invention relates to a method, device and system for determining respiratory information of a subject.

### BACKGROUND OF THE INVENTION

In recent years, the search for non-intrusive methods of monitoring physiological parameters has intensified, particularly in the context of automotive environments. This increased interest is based on the recognition of the limitations associated with traditional physiological monitoring techniques, which often require the use of invasive or uncomfortable sensors. Such sensors can not only prevent continuous monitoring, but also create challenges in confined spaces such as vehicles, where passenger comfort and safety are of primary concern.

The rise of depth sensing technology has revolutionized physiological monitoring by providing a non-contact approach to data acquisition. Widely used in computer vision and gaming applications, depth cameras can capture detailed three-dimensional information about the environment, including the movements and behaviors of objects and people within their field of view. This capability opens up a variety of possibilities for using depth sensing technology in multiple fields, including healthcare and automotive safety.

Among the many applications of depth cameras in physiological monitoring, one area of significant interest is the measurement of respiration. Respiration, the fundamental process of inhaling and exhaling air, serves as a vital indicator of an individual's health status and overall well-being. Monitoring respiratory patterns and rates can provide valuable insight into respiratory conditions, facilitate early detection of disorders, and aid in the assessment of driver fatigue - a critical factor in automotive safety. In addition, understanding respiratory dynamics can contribute to the design of interventions aimed at improving passenger comfort and minimizing the risks associated with long-time vehicle use.

Various approaches to extract respiratory signals and analyze respiratory patterns have been explored. US 2020/138336 A1, for example, discloses a method and an apparatus for determining a breathing status of a person using a depth camera. The method includes acquiring a depth map by photographing one side of a person using a depth camera, extracting a region of the person by separating a background from the acquired depth map, extracting a breathing region from the extracted region of the person, obtaining a depth value for each point of the extracted breathing region for a preset time, and determining a breathing status including a volume of breathing and the number of the breathings by analyzing the obtained depth value. The breathing status of the person can thus be determined in a non-contact manner.

While the use of depth cameras for respiratory measurement is promising, there are several drawbacks and limitations. In particular, current depth cameras struggle to accurately capture respiratory signals in dynamic environments such as vehicles, due to factors including vehicle motion, vibration, and occupant movement. This can lead to inaccuracies and inconsistencies in respiratory measurements, which can affect the reliability of the monitoring system.

Another drawback is that current depth cameras are sensitive to environmental conditions, such as ambient lighting and noisy backgrounds. Changes in lighting conditions within the vehicle cabin, as well as occlusions caused by objects or occupants, can affect the quality of the depth data, and prevent accurate detection of respiratory motion.

Overall, while the use of depth cameras for respiratory measurement has demonstrated potential benefits, addressing the aforementioned disadvantages is critical to achieving the full potential of this technology for improving driver safety and comfort in automotive environments.

### SUMMARY OF THE INVENTION

It is an object of the present invention to improve the reliability and accuracy of the determination of respiratory information of a subject.

In a first aspect of the present invention, a method for determining respiratory information of a subject is presented, the method comprising:
obtaining depth sensor signals relating to distances of a plurality of locations in a region of interest from a depth sensor, wherein the region of interest covers at least a portion of the frontal torso of the subject;
determining a variation in the distances of the plurality of locations in the region of interest based on the depth sensor signals acquired by the depth sensor at a first point in time;
determining an average depth of the plurality of locations in the region of interest based on the depth sensor signals acquired by the depth sensor at the first point in time; and
determining respiratory information of the subject based on the variation and the average depth in the region of interest.

In another aspect, a corresponding device for determining respiratory information of a subject is presented, the device comprising:
a depth sensor input configured to obtain depth sensor signals relating to distances of a plurality of locations in a region of interest from a depth sensor, wherein the region of interest covers at least a portion of the frontal torso of the subject; and
a processor configured to
   - determine a variation in the distances of the plurality of locations in the region of interest based on the depth sensor signals acquired by the depth sensor at a first point in time;
   - determine an average depth of the plurality of locations in the region of interest based on the depth sensor signals acquired by the depth sensor at the first point in time; and
   - determine respiratory information of the subject based on the variation and the average depth in the region of interest.

In a further aspect of the present invention, a system for determining respiratory information of a subject is presented, the system comprising
a depth sensor configured to obtain depth sensor signals relating to distances of a plurality of locations in a region of interest from a depth sensor, wherein the region of interest covers at least a portion of the frontal torso of the subject; and
a device as disclosed herein.

In yet a further aspect of the present invention, there is provided a corresponding computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed device, system, and computer program have similar and/or identical preferred embodiments as the claimed method, in particular as defined in the dependent claims and as disclosed herein.

The present invention is based on the idea that when the person being imaged exhales, the cloth that the person is wearing will be looser around the chest and the depth measurements will already show more variation. At the same time, the average depth will increase as the person's chest moves away from the depth sensor. The opposite effect occurs when the person inhales air. In this case, the chest moves forward (partly because the body is limited at the back by the car seat). The average depth will decrease, and at the same time the clothing will fit a little tighter around the chest. This behavior can be measured by a depth camera and used to improve the determination of respiratory parameters such as respiratory rate, respiratory depth, respiratory volume, inhalation and exhalation of the subject. The method can more effectively separate respiration-induced deformations from other movements, thereby improving the accuracy of respiratory information measurement.

The present invention thus addresses the challenges of known approaches and enables real-time and non-invasive monitoring of respiratory information, e.g. respiration rate and respiration patterns of the driver of a vehicle in an automotive application, thereby enhancing safety, comfort, and overall driving experience.

In an embodiment, the variation is determined by calculating a standard deviation based on the acquired values of the distances of the plurality of locations in the region of interest from the depth sensor. In another embodiment, the variation is determined by first assessing variations in the depth signal at different spatial scales. The spatial scales refer to different levels of detail or resolution within the region of interest. Next, recursive filtering and downscaling steps are applied to the variations. Finally, the variation is determined as a vector representing changes in the depth signal at different spatial scales.

In the context of spatial variation of a spatially varying signal, the spatial scale of the analysis may be useful. A scale space analysis, which involves computing variation at different spatial scales, is a possible approach. This is particularly useful in cases where the exact size of wrinkles in clothing is unknown. In practice, variation can be computed after multiple recursive filter and downscale steps. The result is a vector of variation measurements.

In another preferred embodiment, the determination of the respiratory information of the subject based on the variation and average depth may be implemented as follows. First, a second variation in the distances and a second average depth of the plurality of locations in the region of interest are determined based on the depth sensor signals acquired by the depth sensor at a second point in time that is different from the first point in time. This allows temporal changes in the two parameters to be detected. The proceeding steps entail a comparison of the variation and average depth of the first (initial) point in time with the corresponding parameter determined in the second (subsequent) point in time. Then either an exhalation state with increased second variation and increased second average depth at the second point in time or an inhalation state with decreased second variation and decreased second average depth at the second point in time is assigned, depending on the temporal changes determined in the two parameters. This ensures a more reasonable and accurate approach to the determination of respiratory information.

In yet another embodiment, the determination of the subject's respiratory information based on the variation and the average depth comprises the comparison to predefined thresholds. Specifically, both the variation and the average depth are compared to predefined thresholds of the two parameters. Since the variation corresponds to the number of wrinkles in a person's clothing, it should be highest after a full exhalation. Therefore, if the variation threshold is set at around the average variation value, an exhalation state can be assigned if the variation is above the threshold and an inhalation state can be assigned if the variation is below the threshold. The same applies to average depth. If the average depth threshold is set around the average depth value determined between exhaling and inhaling, the average depth during the exhalation state will be above the threshold as the torso moves away from the depth camera, increasing the average depth. During the inhalation state, the torso moves toward the depth camera, causing the average depth to decrease. Thus, either an inhalation or exhalation state can be assigned based on the current variation and average depth, which is more accurate than using only the average depth.

Another way of determining exhalation and inhalation from the depth sensor signals may involve measuring the movement of the torso compared to the background. Since the torso moves upward during inhalation, the depth sensor signals around the shoulder location will show large temporal variations. In essence, the torso area expands vertically (i.e., in a direction along the longitudinal body axis of the subject's body) during inhalation, which can be detected by the depth sensor signals due to the depth contrast with the background.

In a preferred embodiment, the method according to the invention further comprises considering a reference region, such as the side or ceiling of a car, the headrest of the car, or empty car seats, in the measurements in order to more effectively separate respiration-induced deformations from other movements. First, reference depth sensor signals relating to distances of a plurality of reference locations in a reference region are obtained from the depth sensor. The reference region includes at least a portion of a rigid object exposed to the same external influences as the region of interest. Then, an average reference depth of the plurality of reference locations in the reference region is determined based on the reference depth sensor signals acquired by the depth sensor at the first time. Next, a second average reference depth of the plurality of reference locations in the reference region is determined based on the reference depth sensor signals acquired by the depth sensor at a second time different from the first time. A vibration effect is then determined as the difference between the average reference depth and the second average reference depth, and consequently a corrected average depth is determined as the difference between the second average depth and the vibration effect. Using the variation and the corrected average depth in the region of interest, the subject's respiratory information is determined.

In another preferred embodiment, to further improve the accuracy of the respiratory information detection, the method further comprises the use of camera sensor signals. In particular, image signals of the region of interest acquired over time are first obtained and then combined with the depth sensor signals. Thereafter, feature points in the region of interest are determined based on one or more of i) the obtained image signals, ii) an image generated from the obtained image signals and iii) combined image signals and depth sensor signals. Then, motion information indicative of the temporal motion of the region of interest or of feature points in the region of interest is determined based on the combined image signals and depth sensor signals. Finally, respiration information of the subject is determined based on the variation, the average depth in the region of interest, and the determined motion information. Some feature points determined based on the image data may be rejected based on the depth map, e.g., in case the depth signal a spatial object boundary which indicates that the visual feature may be the effect of occlusion.

In another embodiment the method using camera sensor signals further comprises the following steps. First, feature points in an image generated from the obtained image signals are determined. Then, the 3D position of feature points in the image are determined based on the combined image signals and depth sensor signals. Afterwards, the movement information indicating the temporal movement of the 3D position of the feature points based on the obtained image signals and the obtained depth sensor signals can be determined.

The combination of a color camera sensor with a depth camera sensor can increase the accuracy of the geometric deformation measurements. The idea behind these steps is to add image features to the measurement that allow for an even more accurate estimation of the change in local surface shape. The motion of the image feature combined with the depth variation at the image feature basically provides the 3D geometric change of a localized given point. This is called a 3D vector. If a person happens to be wearing a textured T-shirt, a detailed 3D vector motion field can be generated. By connecting these points, a more accurate volume change related to inhalation or exhalation can be estimated compared to using only the depth map.

The way people breathe varies from person to person and can also vary within a person from time to time. Inhaling and exhaling can be done by the chest and abdomen together. The torso can expand on inhalation and compress on exhalation, so the average depth obtained by the depth camera will be smallest on full inhalation and largest on full exhalation. An algorithm can treat all depths coherently, e.g. combine them (by averaging) into a one-dimensional depth signal, and then extract respiration from that signal.

However, another fairly common breathing pattern is where the chest expands on inhalation (to fill the lungs with air), but the abdomen expands on exhalation. It will be clear that some depths of the upper part of the reference region are smallest when fully inhaled, but in contrast to the above, now the depths of the lower part of the reference region are smallest when fully exhaled. If the depth signals were combined in a simple coherent way, as mentioned above, they could cancel each other out, discarding valuable information and relatively increasing noise.

Therefore, to prevent this misinterpretation of the breathing pattern of this group of people, an embodiment of the invention further proposes that determining an average depth of the plurality of locations in the region of interest includes determining the area of the region of interest from which the depth sensor signals were acquired. This information about the area and/or negative covariance and/or a varying analytic shape fitted to the depth surface is then used in generating the average depth.

The idea of this embodiment is not to combine the multiple depth signals in a simple, coherent way, but to account for the fact that some may be out of phase with others. This can be achieved in different ways, e.g., combining the depths at a low semantic level as raw signals, but also taking into account the negative covariance, or treating the depths separately at a higher semantic level by estimating, based on the geometry, which signals (probably) come from the chest and which from the abdomen.

Negative covariance in this context means that the distance to the abdomen is statistically maximum when the distance to the chest is minimum, or vice versa. Alternatively, a given vertically varying analytic shape (sine function or other) could be fitted to the depth surface as a function of the vertical axis. This shape would then change over time like a wave pattern. This is a more parametric approach, the extreme of which would be to have a geometric human model for which the (chest/abdomen) parameters are estimated in real time.

Respiratory information may include one or more of respiration rate, depth, volume, inhalation, and exhalation of the subject.

As mentioned above, the device according to the present invention comprises, in addition to a processor configured to perform the method for determining respiratory information of a subject described above, a depth sensor input configured to receive, from a depth sensor, depth sensor signals relating to distances of a plurality of locations in a region of interest. Said region of interest includes at least a portion of the frontal torso of said subject. The device may further comprise a color camera sensor input configured to receive color camera sensor signals of the region of interest.

The disclosed system for determining respiratory information of a subject comprises the aforementioned device according to the invention and further a depth sensor configured to obtain depth sensor signals relating to distances of a plurality of locations in a region of interest from a depth sensor. Again, the region of interest includes at least a portion of the frontal torso of the subject. The system may further comprise a color camera sensor configured to obtain color camera sensor signals of the region of interest.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of an embodiment of a system according to the present invention;
Fig. 2 shows a schematic diagram of an embodiment of a device according to the present invention;
Fig. 3A shows a schematic diagram of an embodiment of the system according to the present invention in an inhalation state of a subject;
Fig. 3B shows the schematic diagram of Fig. 3A in an exhalation state of the subject;
Fig. 4A shows a schematic diagram of an embodiment of the system according to the present invention in an inhalation state of a subject with clothing;
Fig. 4B shows the schematic diagram of Fig. 4A in an exhalation state of the subject with clothing;
Fig. 5 shows a flowchart of an embodiment of a method according to the present invention;
Fig. 6A shows a diagram of the average depth during consistent and inconsistent phases;
Fig. 6B shows a diagram of the variation during consistent and inconsistent phases;
Fig. 7 shows a schematic diagram of a depth sensor and color camera sensor combination determining 3D positions of feature points;
Fig. 8A shows a schematic diagram of an embodiment of the system according to the present invention observing a subject in an inhalation state; and
Fig. 8B shows the schematic diagram of Fig. 8A in an exhalation state of the subject.

### DETAILED DESCRIPTION OF EMBODIMENTS

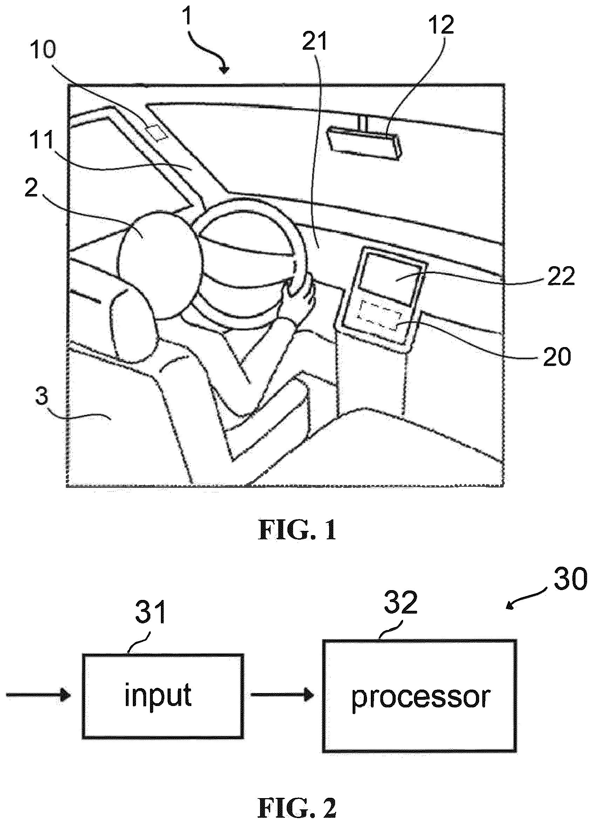

Fig. 1 shows a schematic diagram of an embodiment of a system 1 for determining respiratory information of a subject 2 according to the present invention. In this exemplary embodiment, the system 1 is used for detecting drowsiness or, more generally, the health status or stress status of the driver of a car, for which purpose respiratory information is determined. Respiratory information may preferably be the driver's respiration rate but may additionally or alternatively include one or more of respiration depth, respiration volume, inhalation, and exhalation of the driver 2. Based on the determined respiratory information, it can be determined, for instance, if the driver is still fully awake and attentive or is getting tired and drowsy, in which case an alert may be issued, such as an audible and/or visible signal and/or a haptic signal (e.g. vibration of the steering wheel), or it may be recommended to the driver to make a pause.

The system 1 comprises a depth sensor 10 configured to acquire a depth sensor signals of the subject 2. The depth sensor 10 may, e.g., be a depth camera that is arranged in the A-pillar 11 (as shown in Fig. 1) or in the rear-view mirror 12 so that it can observe the driver 2 from the front, in particular the driver's chest area. Generally, the depth camera can be arranged at any location in the cabin as long as it is able to observe the driver's chest area. The chest depth signal acquired by the depth sensor 10 includes distances between the depth sensor 10 and the subject 2 acquired over time, such as a depth signals, and covers at least part of the subject's chest area.

The system 1 further comprises a device 20 according to the present invention for determining respiratory information of the subject 2 from the depth sensor signals. The device 20 may, e.g., be a processor or computer that is, e.g., arranged somewhere within the dashboard 21. The device 20 may, e.g., be combined with the central processing unit of the car entertainment or car navigation system and may use a central display for issuing warnings or recommendations. In other applications, instead of the car seat 3 supporting the subject 2, there may be a wall or a backrest or another type of back support.

Fig. 2 shows a schematic diagram of an embodiment of a device 30 for determining respiratory information of the subject 2 according to the present invention that may be used as device 20 in the system 1 shown in Fig. 1. The device 30 comprises an input 31 configured to obtain depth sensor signals of the subject 2 and a processor 32 for processing the obtained depth sensor signals and determining the respiratory information. The depth sensor signals may be directly obtained (i.e., received or retrieved) from the depth sensor 10 or from a buffer that temporarily stores the acquired depth sensor signals or from a preprocessor that preprocesses the depth sensor signals acquired by the depth sensor 10.

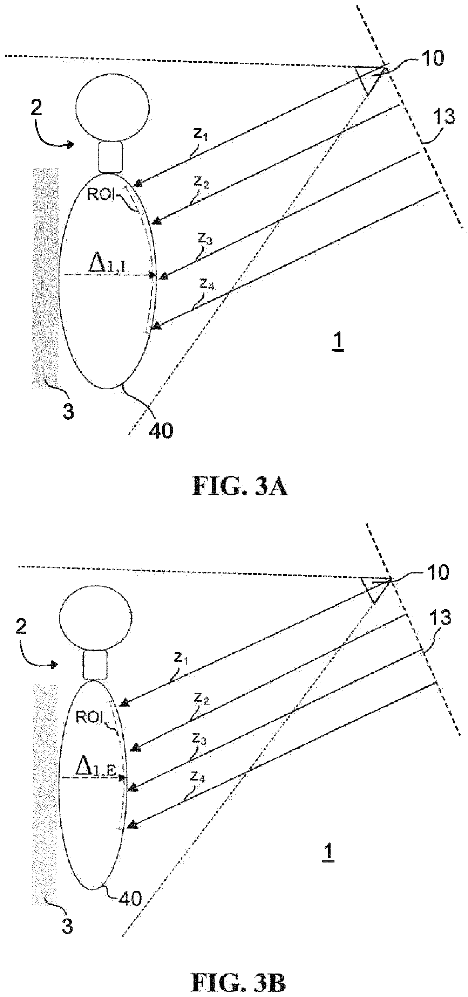

Fig. 3A shows a schematic diagram of an embodiment of the system 1 according to the present invention in an inhalation state of a subject 2 (device 30 not shown). The depth sensor 10 is mounted in front of the subject 2 and directed to its region of interest ROI, a portion of the frontal torso 40 of the subject 2. In this example, four vertically spaced depth measurements *z*₁, *z*₂, *z*₃, *z*₄ are taken, measuring the distance between the sensor plane 13 and the ROI. The inhalation state in Fig. 3A shows an enlarged torso 40 with a horizontal inhalation diameter Δ_{1,I}.

When a person exhales, as shown in Fig. 3B, the torso 40 becomes smaller and assumes an exhalation diameter Δ_{1,E}. Thus, the distance between the sensor plane 13 and the ROI (*z*₁, *z*₂, *z*₃, *z*₄) increases. By averaging these distances, an average depth can be determined that provides information as to whether the subject 2 is in an inhalation or exhalation state. The data can also be used to create a depth map of the region, providing information about the depth and/or spatial relationships within the region. This approach may, however, lead to inaccuracies and inconsistencies in respiratory measurements in applications with noise sources, such as auto motive applications with vibrations or other movements that overlay the respiratory movements of the chest. The present invention thus provides further improvements.

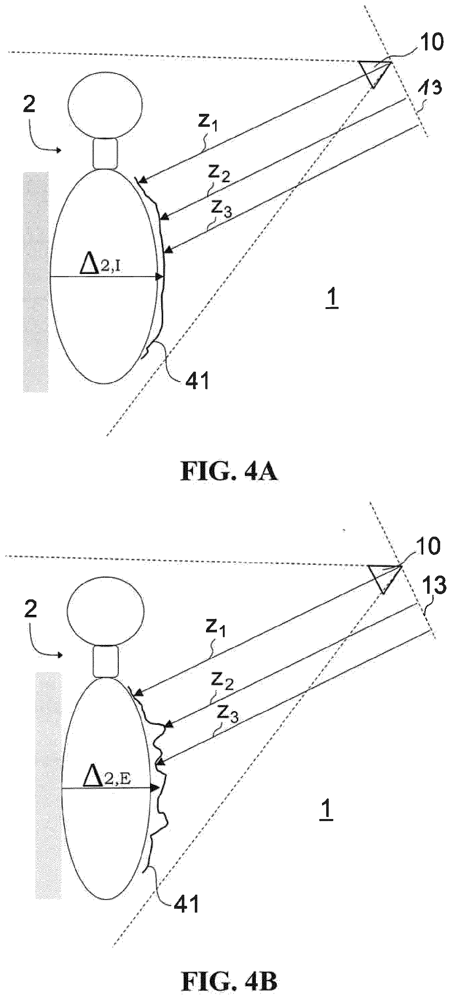

Fig. 4A shows a schematic diagram of an embodiment of the system 1 according to the present invention in an inhalation state of a subject 2 with clothing 41 (device 30 not shown). In this case, the diameter Δ_{2,I} is not limited to the torso 40 alone, but also includes the subject's clothing 41. The depth sensor 10 is capable of measuring not only the depth to the clothing 41 but also the structure of the clothing 41, whether there are many or few wrinkles, here referred to as the variation. This is done by again measuring the distances *z*₁, *z*₂, *z*₃ between the sensor plane 13 and the ROI. In this inhalation state, the clothing 41 on the enlarged torso 40 will be rather smooth and will show little variation in the distances *z*₁, *z*₂, *z*₃.

In the exhalation state shown in Fig. 4B, the torso 40 again decreases in volume, causing a higher amount of wrinkles in the clothing 41. These are detected by the depth sensor 10, since there is a greater variation in the measured distances *z*₁, *z*₂, *z*₃.

The variation may be calculated by analyzing the variation of a signal over space, where the size of the area under consideration is a determining factor. A method known as scale space analysis is often used, where the signal is examined at different scales or sizes. This approach is advantageous in situations where the exact dimensions of wrinkles in clothing are unknown. In particular, the changes observed after repeated filtering and downscaling of the signal are quantified. The result is a set of measurements that illustrate the variation of the signal at different scales.

In general, when the person is exhaling, the cloth that the person is wearing will be looser around the chest and the average depth measurements will show more variation. At the same time, the average depth will increase as the person's chest moves away from the depth sensor 10. The opposite effect occurs when the person inhales air. In this case, the chest moves forward (partly because the body is limited at the back by the back support 5). The average depth will decrease and at the same time the clothing 41 will fit a little tighter around the chest. The invention proposes to use temporal changes in surface geometry measured by a depth camera sensor 2 to characterize inhalation and exhalation.

The system 1 can be used in cars where the depth camera is mounted such that it images, for example, a driver from the front. There, the depth map contains the distance as measured from the camera viewpoint and typically (but not necessarily) has a size of 640x480 pixels, for example, when using a Time of Flight (ToF) depth camera.

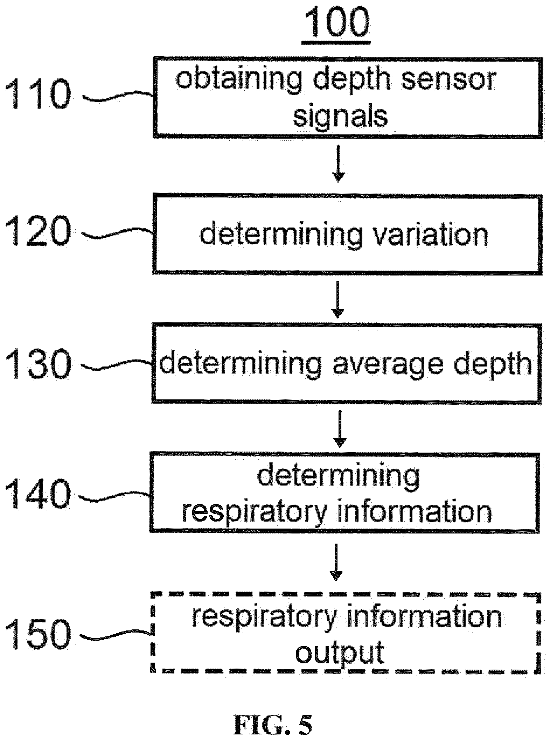

Fig. 5 illustrates a flowchart of an embodiment of a method 100 according to the present invention. In a first step 110 of the method 100 depth sensor signals relating to distances of a plurality of locations in a region of interest ROI from the depth sensor 10 or sensor plane 13. The region of interest ROI includes at least a portion of the frontal torso 40 of the subject 2, particularly the chest region, since the periodic changes during breathing are most pronounced in this area. The distances may include those shown in Figs. 3A, 3B, 4A and 4B.

In a second step 120, a variation in the distances of the plurality of locations in the region of interest is determined as described above. This determination is based on the depth sensor signals that have been acquired by the depth sensor 10 at a first point in time. In a subsequent or simultaneous step 130, an average depth of the plurality of locations in the region of interest is determined. This is based on the same depth sensor signals that were acquired by the depth sensor 10 at the first point in time. The final step 140 is to determine respiratory information of the subject 2 based on the previously determined variation and the average depth in the region of interest.

Optionally, the method 100 may further comprise a step 150 of providing the determined respiratory information as an output in the form of a display or transferring the information to another system that bases its functions on said respiratory information.

The respiratory information, as mentioned above, can be obtained from the information on the change between the inhalation and exhalation state of the subject 2, but can also include further information on the respiratory rate, respiratory depth and/or respiratory volume based on the determined variation and average depth.

The above analysis models an ideal situation. In reality, people may wear loose clothing, and in this case the topology of the measured surface will move, but the surface will not always be significantly smoother. However, the average depth will still decrease during inhalation.

It will also make a difference if a person is wearing a t-shirt or a coat. In the latter case, interpretation of roughness changes will not work. Therefore, it may be useful to classify the type of clothing 41 a person is wearing based on a combination of the depth map and the image data.

By simultaneously measuring both global depth changes (average depth) and local changes in tissue structure (variation), respiration-induced deformations can be separated from other movements.

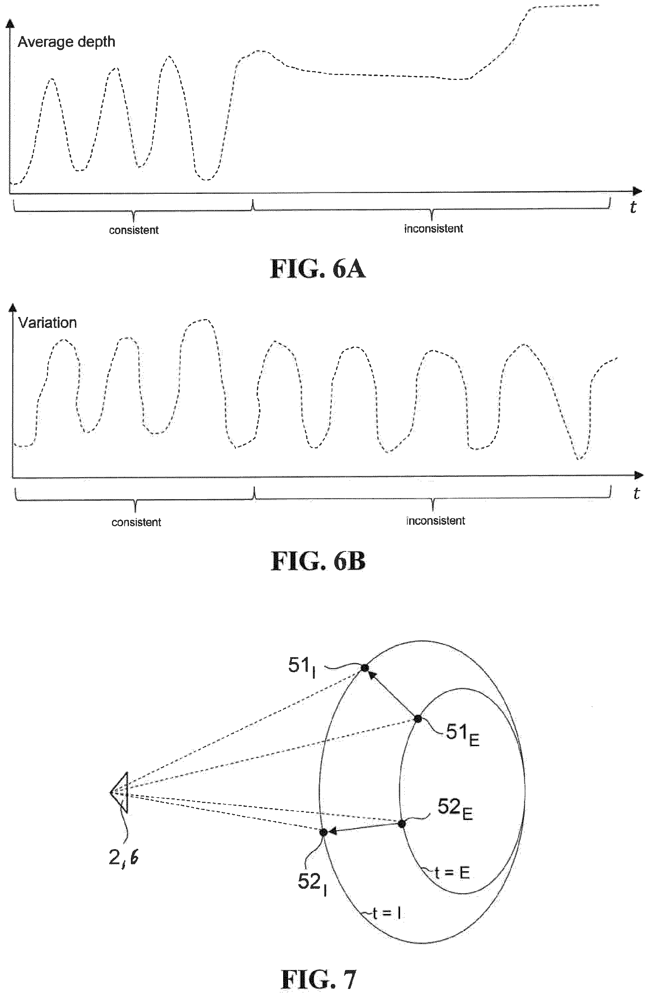

Figs. 6A and 6B show schematic plots of average depth (Fig. 6A) and variation (Fig. 6B) during steady and consistent, and unsteady and inconsistent phases. It illustrates how respiration-induced deformations can be separated from other motions using hypothetical signals. In the first part of the time axis, global depth changes and local (depth) structure changes are consistent, i.e., they change synchronously, either in phase or out of phase. This is not true for the second part of the time axis, where the cloth structure change is still periodic, but the global depth change is not. Combining the two cues therefore has the potential to improve robustness.

For example, such a signal can occur when the subject 2 intentionally moves toward the depth sensor 10 or when environmental factors such as vibration or driving over uneven surfaces are encountered. Accurate determination of respiratory information is still possible, making the system more reliable.

Another approach to improving the reliability of the system 1 is to introduce a reference plane. For example, when the depth sensor 10 is mounted in a vehicle, vibration can cause a global displacement of the measured average depth. This global displacement can be measured on rigid objects such as the side or ceiling of the car, the headrest of the car, or empty car seats. This temporal vibration effect can then be subtracted from the average depth determined before breathing information is extracted.

Fig. 7 shows a schematic diagram of a depth sensor 10 and color camera sensor 6 combination determining 3D positions of feature points in the region of interest ROI. The combination of a color camera sensor 6 with a depth camera sensor 2 can increase the accuracy of the geometric deformation measurements. This may be done by performing image feature point detection in the color image over time and establishing a correspondence of these feature points. When these 2D image feature points are combined with the depth sensor 10, the 3D position of the feature points can be estimated, and the temporal movement of these 3D feature points can be determined. Especially the lateral movement (e.g., expansion) of the chest over time can then be measured more accurately compared with the situation that only a depth sensor 10 is used. This arrangement is illustrated in Fig. 7. Furthermore, when registered, the 2D image can help to improve the accuracy of the region of interest (chest) detection in the depth image, e.g., by detecting the shoulders. Tracking the local displacements within the monochrome or color image provides an additional cue to separate respiration-induced deformations from other movements.

In the example shown in Fig. 7, the image feature points 51_{E} and 52_{E} are detected during the exhalation state t = E of a subject 2 by both the color camera 6 and the depth sensor 10. Then, image feature points 51_{I} and 52_{I} are detected during the inhalation state t = I. By combining the two types of information on these feature points, an accurate 3D position of the feature points can be determined.

The way people breathe may vary within a person or from person to person. Inhalation and exhalation may be performed by the chest and abdomen together, with the torso 40 as a whole expanding on inhalation and compressing on exhalation (Δ_{1,I} > Δ_{1,E}), see Figs. 3A and 3B. Consequently, the depths (*z*₁, *z*₃, *z*₃, *z*₄), observed by the depth camera, will be smallest when fully inhaled and largest when fully exhaled. Thus, an algorithm can treat all depths (*z*₁, *z*₃, *z*₃, *z*₄) coherently, averaging them into a one-dimensional depth signal, and then extract respiration from that signal.

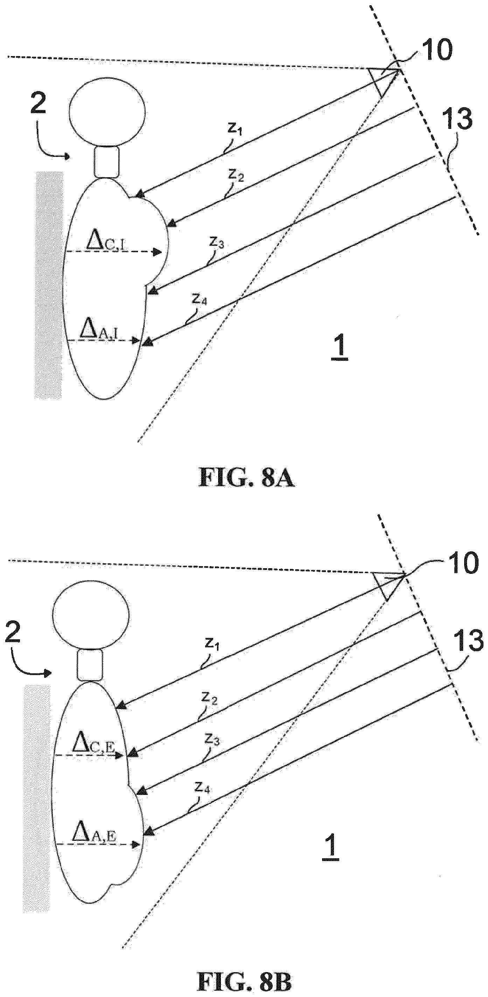

Fig. 8A shows a schematic of an embodiment of the system 1 according to the present invention of a subject 2 in an inhalation state (device 30 not shown). In this other fairly common breathing pattern, the chest expands during inhalation, but the abdomen expands during exhalation. As a result, the diameter of the chest during inhalation Δ_{C,I} is increased, and the depths *z*₁ and *z*₂ are smallest when fully inhaled. However, opposite is true for the abdominal part of the subject 2. The diameter Δ_{A,I} is decreased and the depths *z*₃ and *z*₄ are greatest at complete inspiration.

Fig. 8B shows the schematic diagram of Fig. 8A in an exhalation state. In contrast to the above, Δ_{C,E} is decreased and Δ_{A,E} is increased. Thus, *z*₁ and *z*₂ are largest and *z*₃ and *z*₄ are smallest at full exhalation. If the depth signals were combined in a simple coherent way, as mentioned previously, they would cancel each other out, discarding valuable information and relatively increasing noise. In general, the chest expands during inhalation, compresses during exhalation (Δ_{C,I} > Δ_{C,E}) and the abdomen compresses during inhalation, expands during exhalation (Δ_{A,I} < Δ_{A,E}).

In summary, the present invention allows a more robust and accurate determination of respiratory information by exploiting temporal changes in surface geometry as measured by a depth camera sensor to characterize inhalation and exhalation.

The invention can be used in micro-motion vital sign measurements in various applications, including ambulatory applications and automotive applications to monitor the condition of the driver.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Method for determining respiratory information of a subject, the method comprising:
obtaining depth sensor signals relating to distances of a plurality of locations in a region of interest from a depth sensor, wherein the region of interest covers at least a portion of the frontal torso of the subject;
determining a variation in the distances of the plurality of locations in the region of interest based on the depth sensor signals acquired by the depth sensor at a first point in time;
determining an average depth of the plurality of locations in the region of interest based on the depth sensor signals acquired by the depth sensor at the first point in time; and
determining respiratory information of the subject based on the variation and the average depth in the region of interest.

2. Method according to claim 1, wherein the variation is determined by computing a standard deviation of the obtained distances of the plurality of locations in the region of interest.

3. Method according to claim 1, wherein the variation is determined by:
assessing variations in the depth signal at different spatial scales, wherein the spatial scales refer to varying levels of detail or resolution within the region of interest;
applying recursive filters and downscaling steps to the variations;
determining the variation as a vector representing changes in the depth signal across different spatial scales.

4. Method according to any preceding claim, wherein determining respiratory information of the subject based on the variation and the average depth comprises:
determining a second variation in the distances and a second average depth of the plurality of locations in the region of interest based on the depth sensor signals acquired by the depth sensor at a second point in time different from the first point in time;
comparing the variation to the second variation;
comparing the average depth to the second average depth;
assigning an exhalation state with increased second variation and increased second average depth at the second point in time; and
assigning an inhalation state with reduced second variation and reduced second average depth at the second point in time.

5. Method according to any preceding claim, wherein determining respiratory information of the subject based on the variation and average depth comprises:
comparing the variation to a predefined variation threshold;
comparing the average depth to a predefined depth threshold;
assigning an exhalation state to the subject if the variation is above the variation threshold and the average depth is above the depth threshold; and
assigning an inhalation state to the subject if the variation is below the variation threshold and the average depth is below the depth threshold.

6. Method according to claim 4, further comprising:
obtaining reference depth sensor signals relating to distances of a plurality of reference locations in a reference region from the depth sensor, wherein the reference region covers at least a portion of a rigid object exposed to the same external influences as the region of interest;
determining an average reference depth of the plurality of reference locations in the reference region based on the reference depth sensor signals acquired by the depth sensor at the first point in time; and
determining a second average reference depth of the plurality of reference locations in the reference region based on the reference depth sensor signals acquired by the depth sensor at a second point in time different from the first point in time;
determining a vibration effect as the difference between the average reference depth and the second average reference depth; and
determining a corrected average depth as the difference between the second average depth and the vibration effect; and
using the variation and the corrected average depth in the region of interest for determining respiratory information of the subject.

7. Method according to any preceding claim, further comprising:
obtaining image signals of the region of interest acquired over time;
combining the image signals and the depth sensor signals;
determining feature points in the region of interest based on one or more of i) the obtained image signals, ii) an image generated from the obtained image signals and iii) combined image signals and depth sensor signals;
determining movement information indicating the temporal movement of the region of interest or of feature points in the region of interest based on the combined image signals and depth sensor signals; and
determining respiratory information of the subject based on the variation, the average depth in the region of interest and the determined movement information.

8. Method according to claim 7, further comprising:
determining feature points in an image generated from the obtained image signals;
determining the 3D position of feature points in the image based on the obtained image signals and the obtained depth sensor signals; and
determining the movement information indicating the temporal movement of the 3D position of the feature points based on the combined image signals and depth sensor signals.

9. Method according to any preceding claim,
wherein determining an average depth of the plurality of locations in the region of interest includes determining the area of the region of interest from which the depth sensor signals have been acquired and using, in generating the average depth, this information about the area and/or negative covariance and/or a varying analytic shape fitted to the depth surface.

10. Method according to any one of the preceding claims, wherein the respiratory information comprises one or more of respiration rate, respiration depth, respiratory volume, inhalation, and exhalation of the subject.

11. Device for determining respiratory information of a subject, the device comprising:
a depth sensor input configured to obtain depth sensor signals relating to distances of a plurality of locations in a region of interest from a depth sensor, wherein the region of interest covers at least a portion of the frontal torso of the subject; and
a processor configured to
- determine a variation in the distances of the plurality of locations in the region of interest based on the depth sensor signals acquired by the depth sensor at a first point in time;
- determine an average depth of the plurality of locations in the region of interest based on the depth sensor signals acquired by the depth sensor at the first point in time; and
- determine respiratory information of the subject based on the variation and the average depth in the region of interest.

12. Device according to claim 11, further comprising:
a color camera sensor input configured to obtain color camera sensor signals of the region of interest.

13. System for determining respiratory information of a subject, the system comprising
a depth sensor configured to obtain depth sensor signals relating to distances of a plurality of locations in a region of interest from a depth sensor, wherein the region of interest covers at least a portion of the frontal torso of the subject; and
a device as claimed in claim 10.

14. System according to claim 13,
further comprising a color camera sensor configured to obtain color camera sensor signals of the region of interest.

15. Computer program comprising program code means for causing a computer to carry out the steps of the method as claimed in any one of claims 1 to 10 when said computer program is carried out on the computer.
